# EUROPEAN PATENT APPLICATION

(11) **EP 4 752 131 A1**
(43) Date of publication of application: **03.06.2026**
(21) Application number: 24216189.1
(22) Date of filing: 28.11.2024
(51) Int. Cl.: C07C 69/738, C07D 311/76

(54) **PROCESS TO OBTAIN SUBSTITUTED CYCLOHEXENES AND DERIVATIVES THEREFROM AS INTERMEDIATES IN THE PREPARATION OF FUNGICIDES**

(71) Applicant: BASF SE, 67056 Ludwigshafen am Rhein (DE)
(72) Inventor: BORATE, Kailaskumar, Navi Mumbai, Maharashtra 400705 (IN); THOMBAL, Raju, Newasa, 414607 (IN); KADUSKAR, Rahul, Navi Mumbai, Maharashtra 400705 (IN); SHYMANSKA,Nataliia, 67056 Ludwigshafen am Rhein (DE); PATIL, Bhamesh, Navi Mumbai, Maharashtra 400705 (IN); RAUT, Dhanyakumar, Navi Mumbai, Maharashtra 400705 (IN); DHANAWATE, Ram, Navi Mumbai, Maharashtra 400705 (IN); SHINDE, Harish, Navi Mumbai, Maharashtra 400705 (IN); GOETZ, Roland, 67056 Ludwigshafen am Rhein (DE)
(74) Representative: BASF IP Association

(57) **Abstract**

The present invention relates to a process for preparation of substituted cyclohexenes as intermediates for the preparation of substituted enol ether fungicides. The invention also relates to processes for the preparation of novel intermediates of enol ether fungicide synthesis starting from substituted cyclohexenes.

## Description

The present invention relates to a process for preparation of substituted cyclohexenes as intermediates for the preparation of substituted enol ether fungicides. The invention also relates to processes for the preparation of novel intermediates of enol ether fungicide synthesis starting from substituted cyclohexenes.

Enol ether fungicides are known from WO2021/153754, WO2021/219386, WO2021/219387, WO2021/219388, WO2021/219390, WO2021/249928, WO2022/008303, WO2022/013009, WO2022//03390, WO2023/072670, WO2023/072671, WO2023/072672, and EP4242198. The preparation of such fungicidal compounds was described in the abovementioned references by a coupling of the intermediates of the formula Int-I 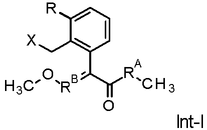 inter alia with substituted benzaldehyde oxime compounds. It has been known from IPCOM000274066D that intermediates of the type Int-I can be prepared from 8-methyl-isochroman-3-one Int-II.

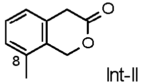

The preparation of isochroman-3-ones is known from EP0906304 B1 and involves a benzylic halogenation step. The corresponding preparation of 8-substituted isochroman-3-ones via benzylic halogenation lacks efficiency and/or suffers from selectivity issues leading to formation of side-products.

Therefore, new methods are desirable for preparing 8-substituted isochroman-3-ones and precursors thereof as synthetic intermediates for fungicides. Thus, it was an object of the present invention to overcome the disadvantages of known processes and to provide improved, more economical processes to access such compounds.

The synthesis of a differently substituted cyclohexene derivative under cryogenic conditions has been described in IN2014DE02082A:

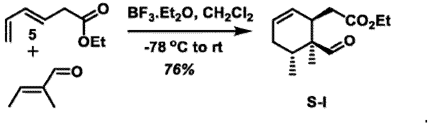

The process according to the present invention differs from the process described in IN2014DE02082A in the substitution pattern of the compound of formula I, and surprisingly overcomes the disadvantage of the use of cryogenic conditions while enabling a cost-efficient preparation of cyclohexenes at high yield.

Accordingly, the present invention relates to a process for preparing compounds of formula I 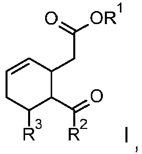 in which
R¹ is H, C₁-C₁₂-alkyl, phenyl, C₁-C₄-alkyl-phenyl, or C₁-C₄-alkyl-C₃-C₆-cycloalkyl, wherein phenyl rings are unsubstituted or substituted with one halogen;
R² is H or C₁-C₆-alkyl; and
R³ is C₁-C₆-alkyl, C₂-C₄-alkenyl, C₃-C₆-cycloalkyl or C₁-C₄-alkoxy, wherein R³ is unsubstituted or substituted with 1, 2, 3, 4 or 5 halogens;
the process comprising
   (i) mixing a compound of formula III 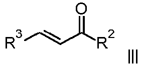
      in which R² and R³ are as defined above for formula I,
      with a compound of formula II 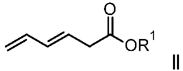
      in which R¹ is as defined above for formula I;
   (ii) adding boron trifluoride diethyl etherate;
   (iii) stirring the mixture;
characterized by the fact that the temperature is between -20°C and 80°C.

The abovementioned temperature range can be preferably between -10°C to 40°C or even more preferably between 0°C to 25°C. In another embodiment, the abovementioned temperature range can be defined for each of the reaction steps (i) to (iii) as follows:
A preferred temperature range for step (i) is from -10°C to 40°C, more preferably from 0°C to 30°C, even more preferably from 10°C to 25°C.

A preferred temperature range for step (ii) is from -20°C to 30°C, more preferably from -10°C to 25°C, even more preferably from -5°C to 10°C.

A preferred temperature range for step (iii) is from -20°C to 80°C, more preferably from -10°C to 50°C, even more preferably from 0°C to 30°C.

Preferably, the reaction takes place in presence of a solvent.

Even more preferably, compound II and/or compound III are employed as solution in a suitable solvent.

Suitable solvents are, in general, aromatic hydrocarbons such as toluene, o-, m- and p-xylene, cymene, cumene; halogenated hydrocarbons such as chlorobenzene, dichlorobenzene, dichloromethane (DCM), dichloroethane; ethers such as diethyl ether, dioxane, tetrahydrofuran (THF), 2-Me-THF, cyclopentyl methyl ether, diisopropyl ether, diphenyl ether; nitriles such as acetonitrile and propionitrile; alcohols such as methanol, ethanol, n-propanol, isopropyl alcohol, n-butanol, tert-butanol; amidic solvents such as dimethylformamide (DMF), dimethyl acetamide, N-methyl-2-pyrrolidone (NMP), N-ethyl-2-pyrrolidone (NEP); sulfoxide solvents such as dimethyl sulfoxide (DMSO), sulfone solvents such as sulfolane; esters such as ethyl acetate, isopropyl acetate. It is also possible to use mixtures of the solvents mentioned. Preferred are halogenated hydrocarbons and ether solvents. More preferred are DCM, dichloroethane, and diethyl ether. Particularly preferred are DCM and diethyl ether.

The concentration of compound II at step (i) is preferably 0.3 to 5 mol/L, more preferably 0.4 to 2.0 mol/L, even more preferably 0.5 to 1.0 mol/L.

The amount of compound III relative to compound II is preferably in the range from 1.0 to 5.0 molar equivalents, more preferably 1.0 to 3.0 molar equivalents, even more preferably from 1.0 to 1.5 molar equivalents.

The amount of boron trifluoride diethyl etherate (BF₃·OEt₂) relative to compound II is preferably in the range from 0.01 to 3.0 molar equivalents, more preferably from 0.05 to 1.0 molar equivalents, even more preferably from 0.1 to 0.5 molar equivalents.

BF₃·OEt₂ can be added undiluted or as a solution in one or more of the abovementioned solvents. In one embodiment, BF₃·OEt₂ is added undiluted. In another embodiment, BF₃·OEt₂ is added as a solution. Preferably, the solvent is DCM, monochlorobenzene, toluene, xylene and/or diethyl ether. Preferably, the concentration of the solution is in the range from 10 to 50 mol/L.

The duration of step (ii) is preferably between 5 min and 2 h, more preferably between 10 min and 90 min, even more preferably between 20 min and 70 min. The duration of step (iii) is not particularly limited and typically it is in the range of 1 h to 24 h, preferably from 2 h to 20 h, even more preferably it is approximately 16 h.

Optionally, the reaction can be stopped after step (iii) by quenching. Suitable quenching agents are, in general, aqueous solutions of inorganic salts such as sodium bicarbonate, sodium carbonate, sodium hydroxide, potassium hydroxide, ammonium hydroxide, ammonium chloride, preferably sodium bicarbonate.

According to one embodiment, the reaction mixture can be used directly in the next reaction.

According to another embodiment, the solvent can be removed from the reaction mixture by usual means (e.g. washing drying and concentrating *in vacuo*) to obtain the desired compound I. Compounds I can be present as different stereoisomers.

Compounds II and III are commercially available or can be obtained according to methods described in the literature (e.g., T. R. Hoye et al., Synthetic Communications 1982, 12, 183-187 and K. S. Kim et al., Synthesis 1987, 11, 1017-1018).

Further, the compound I may be treated with a reducing agent to yield a compound IV 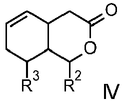 in which R² and R³ are as defined above.

Generally, the process to obtain compounds IV is carried out at temperatures from -10°C to 120°C. A preferred temperature range is from 0°C to 100°C, more preferably from 0°C to 60°C, even more preferably from 0°C to 25°C.

Preferably, the reaction takes place in presence of a solvent.

Suitable solvents are, in general, aromatic hydrocarbons such as toluene, o-, m- and p-xylene, cymene, cumene; halogenated hydrocarbons such as chlorobenzene, dichlorobenzene, dichloromethane, dichloroethane; ethers such as dioxane, THF, 2-Me-THF, cyclopentyl methyl ether, diisopropyl ether, diphenyl ether; alcohols such as methanol, ethanol, n-propanol, isopropyl alcohol, n-butanol, tert-butanol; water and aqueous solutions of inorganic bases such as sodium hydroxide, potassium hydroxide, sodium carbonate, potassium carbonate. It is also possible to use mixtures of the solvents mentioned. Preferred are ether and alcohol solvents. More preferred are ethanol, methanol, THF. Even more preferred is ethanol.

Suitable reducing agents are, in general, stoichiometric reductants such as NaBH₄, NaCNBH₃, LiAlH₄, LiBH₄, Et₃SiH/trifluoroacetic acid (TFA), Sml₂, and metal borides, such as Nickel/Cobalt borides. NaBH₄ can be used alone or in combination with a Lewis acid (for example, NaBH₄/CeCl₃) or in combination with a base (for example, NaBH₄/NaOH). Further suitable reducing agents comprise a metal catalyst in the presence of a terminal reductant such as hydrogen (H₂), for example as described in Pietropaolo et al, Appl. Catal. A. Gen. 2010 p. 77 and references therein, or transfer hydrogenations such as those using Pd/C, NEt₃/HCO₂H. Preferably NaBH₄ or NaCNBH₃ are employed, more preferably NaBH₄ is employed.

The reducing agent may be employed neat or in solution. The reducing agent may be employed in stoichiometric amounts or in excess relative to the compound IV. Preferably between 1.0 and 3.0 molar equivalents of reducing agent are employed relative to compound IV, more preferably 1.5 molar equivalents of reducing agent are employed relative to compound IV.

The reaction period after addition of the reducing agent is not particularly limited and typically it is in the range of 30 min to 6 h, preferably from 1 h to 3 h.

Optionally, the reaction can be stopped by quenching. Suitable quenching agents are, in general, aqueous solutions such as aqueous hydrochloric acid or aqueous ammonium chloride, preferably aqueous hydrochloric acid.

The compound IV may be treated with an oxidizing agent to yield an 8-substituted isochroman-3-one compound V 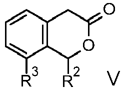 in which R² and R³ are as defined above.

Generally, the process step to obtain compounds V is carried out at temperatures from 50°C to 300°C. A preferred temperature range is from 50°C to 180°C, more preferably from 100°C to 160°C, even more preferably at about 150°C.

Preferably, the reaction takes place in presence of a solvent.

Suitable solvents are, in general, aromatic hydrocarbons such as toluene, o-, m- and p-xylene, cymene, cumene, nitrobenzene; halogenated hydrocarbons such as chlorobenzene, dichlorobenzene, dichloroethane; ethers such as dioxane, THF, 2-Me-THF, anisole, diphenyl ether; nitriles such as acetonitrile and propionitrile; alcohols such as ethanol, n-propanol, isopropyl alcohol, n-butanol, tert-butanol; amidic solvents such as DMF, dimethylacetamide, NMP, NEP; sulfoxide solvents such as DMSO, sulfone solvents such as sulfolane; esters such as ethyl acetate, isopropyl acetate. It is also possible to use mixtures of the solvents mentioned. Preferred are high-boiling solvents such as xylene, cumene, chlorobenzene, dichlorobenzene, DMSO, DMF, NMP. More preferred is diphenyl ether.

Suitable oxidizing agents are, in general, stoichiometric oxidants such as 4,5-dichloro-3,6-dioxocyclohexa-1,4-diene-1,2-dicarbonitrile (DDQ), oxygen, chloranil, (2,2,6,6-tetramethylpiperidin-1-yl)oxyl (TEMPO), tert-butyl hydroperoxide, sulfur. Further suitable oxidizing agents comprise a metal catalyst generally in the presence of a terminal oxidant.

It may be advantageous to employ an additive in the process step to obtain compounds V. Suitable additives are nitrobenzenes, alkenes (including styrenes), and aldehydes (including benzaldehydes). Preferably, nitrobenzene is employed.

The reaction period after addition of the oxidizing agent is not particularly limited and typically it is in the range of 12 to 24 h.

The reaction mixtures are worked up in a customary manner, for example by mixing with water, separating the phases and, if appropriate, chromatographic purification of the crude products. In some cases, the intermediates and end products are obtained in the form of colorless or slightly viscous oils which can be freed from volatile components or purified under reduced pressure and at moderately elevated temperatures. If the intermediates and end products are obtained as solids, purification can also be carried out by recrystallization or digestion.

In addition, the invention also relates to novel compounds of formula I 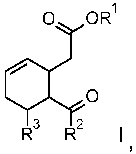
compounds of formula IV 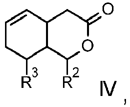
and compounds of formula V 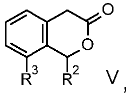
in which R¹, R², and R³ are as defined herein.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. It is to be understood that the term "comprising" is not limiting. For the purposes of the present invention the term "consisting of" is considered to be a preferred embodiment of the term "comprising".

Unless otherwise indicated, the following definitions are set forth to illustrate and define the meaning and scope of the various terms used to describe the invention herein and the appended claims. These definitions should not be interpreted in the literal sense as they are not intended to be general definitions and are relevant only for this application.

The term "compounds I" refers to compounds of formula I. Likewise, this terminology applies to all sub-formulae, e. g. "compounds II" refers to compounds of formula II or "compounds V" refers to compounds of formula V, etc.

The term "independently" when used in the context of selection of substituents for a variable, it means that where more than one substituent is selected from a number of possible substituents, those substituents may be the same or different.

The organic moieties or groups mentioned in the above definitions of the variables are collective terms for individual listings of the individual group members. The term "Cᵥ-C_{w}" indicates the number of carbon atom possible in each case.

The term "halogen" refers to fluorine, chlorine, bromine and iodine.

The term "C₁-C₁₂-alkyl" refers to a straight-chained or branched saturated hydrocarbon group having 1 to 12 carbon atoms, for example, methyl (CH₃), ethyl (C₂H₅), propyl, 1-methylethyl (isopropyl), butyl, 1-methylpropyl, 2-methylpropyl, 1,1-dimethylethyl, heptyl, decanyl, dodecanyl.

The term "C₂-C₄-alkenyl" refers to a straight-chain or branched unsaturated hydrocarbon radical having 2 to 4 carbon atoms and a double bond in any position such as ethenyl, 1-propenyl, 2-propenyl, 1-methylethenyl, 1 butenyl, 2-butenyl, 3-butenyl, 1-methyl-1-propenyl, 2-methyl-1-propenyl, 1-methyl-2-propenyl, 2-methyl-2 propenyl.

The term "C₁-C₄-alkoxy" refers to a straight-chain or branched alkyl group having 1 to 4 carbon atoms which is bonded via an oxygen, at any position in the alkyl group, e.g. OCH₃, OCH₂CH₃, O(CH₂)₂CH₃, 1-methylethoxy, O(CH₂)₃CH₃, 1-methylpropoxy, 2-methylpropoxy or 1,1-dimethylethoxy.

The term "C₃-C₆-cycloalkyl" refers to monocyclic saturated hydrocarbon radicals having 3 to 6 carbon ring members, such as cyclopropyl (C₃H₅), cyclobutyl, cyclopentyl or cyclohexyl.

The term "C₁-C₄-alkyl-phenyl" refers to a straight-chained or branched saturated hydrocarbon group having 1 to 4 carbon atoms and carrying a phenyl substituent, for example, benzyl (-CH₂-C₆H₅), homobenzyl (-CH₂CH₂-C₆H₅), 1-phenylethyl (-CH(CH₃)-C₆H₅).

The term "C₁-C₄-alkyl-C₃-C₆-cycloalkyl" refers to a straight-chained or branched saturated hydrocarbon group having 1 to 4 carbon atoms and carrying a C₃-C₆-cycloalkyl substituent, for example, -CH₂-cyclopropyl, -CH₂CH₂-cyclobutyl, -CH₂-cyclohexyl.

In respect of the variables, the embodiments of the compounds II, III, IV and V correspond to the embodiments of the compounds I.

Preference is given to those compounds I and where applicable also to compounds II, III, IV and V, wherein the substituents and variables (such as R¹, R², R³) have independently of each other or more preferably in combination (any possible combination of 2 or more substituents as defined herein) the following meanings:
According to one embodiment, the substituent R¹ is unsubstituted C₁-C₆-alkyl, more preferably methyl or ethyl, in particular ethyl.

Preferably, R² is H.

Preferably, R³ is unsubstituted C₁-C₆-alkyl, more preferably methyl or ethyl, in particular methyl.

According to one embodiment, the substituent R¹ in formula I is unsubstituted C₁-C₆-alkyl, more preferably methyl or ethyl, in particular ethyl.

Preference is given to those compounds I, IV, and V, wherein the substituents R² and R³ have independently of each other or more preferably in combination (any possible combination of 2 or more substituents as defined herein) the following meanings:
According to one embodiment, R² is H or C₁-C₃-alkyl, more preferably H or methyl, in particular H.

According to one embodiment, R³ is unsubstituted C₁-C₆-alkyl, more preferably methyl or ethyl, in particular ethyl.

Preferably, compound I is ethyl 2-(6-formyl-5-methyl-cyclohex-2-en-1-yl) acetate:

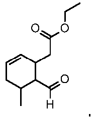

Preferably, compound IV is 8-methyl-1,4,4a,7,8,8a-hexahydroisochromen-3-one:

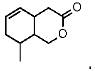

Preferably, compound V is 8-methylisochroman-3-one:

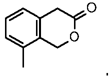

Further, the invention relates to the use of compounds I as defined herein for the preparation of compounds V as defined herein.

### Examples

### Example 1: Preparation of ethyl 2-(6-formyl-5-methyl-cyclohex-2-en-1-yl)acetate

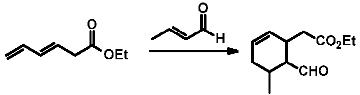

DCM (1500 mL) was charged into the ethyl (3E)-3,5-hexadienoate (502 g, 3.587 mol) in the 20 L reactor followed by the addition of crotonaldehyde (301.76 g, 4.305 mol). The reaction mixture was cooled to 0°C and 50% BF₃·OEt₂ solution in diethyl ether (254.31 g, 0.896 mol) solution diluted with DCM (1000 mL) added dropwise over approximately 60 min at 0°C. After completion of addition, the reaction mixture was further stirred reaction at 25°C for 16 h. After completion of the reaction, the reaction mass was quenched using saturated solution of sodium bicarbonate at 0° within 40 min. Separated the organic phase and aqueous layer was extracted using DCM (500 mL). Organic layer was washed with brine (500 ml), dried over sodium sulphate, and evaporated to obtain ethyl 2-(6-formyl-5-methyl-cyclohex-2-en-1-yl) acetate (640 g; 85% yield).

¹H NMR (500 MHz, Chloroform-d) δ 9.89 - 9.52 (m, 1H), 5.84 - 5.47 (m, 2H), 4.14 (qd, J = 7.1, 1.3 Hz, 3H), 3.04 - 2.92 (m, 1H), 2.55 - 2.37 (m, 3H), 2.36 - 2.13 (m, 3H), 1.90 (dddt, J= 15.5, 5.9, 4.1, 2.3 Hz, 1H), 1.76 (dddd, *J =* 18.1, 7.4, 3.6, 2.0 Hz, 1H), 1.31 - 1.21 (m, 3H), 1.05 (dd, J= 19.2, 6.6 Hz, 4H).

### GCMS Method

| | | | | |
|---|---|---|---|---|
| Total Flow: | 35.4 mL/min | Oven Temp. Program: | | |
| Column Flow: | 1.04 mL/min | Rate (°C/min) | Temp. (°C) | Hold Time (min) |
| Linear Velocity: | 36.8 cm/sec | - | 40.0 | 2.00 |
| Purge Flow: | 3.0 mL/min | 20.00 | 250.0 | 0.00 |
| Split Ratio: | 30.0 | 50.00 | 280.0 | 2.00 |
| Column: | Rxi-5Sil MS, 30m, 0.25mm ID, 0.25µm | | | |

### Ethyl (3E)-3,5-hexadienoate: 6.38 min

Ethyl 2-(6-formyl-5-methyl-cyclohex-2-en-1-yl) acetate as a mixture of diastereoisomers: 10.17 & 10.25 min

Following the procedure described above with variation of the reaction temperature at steps (ii) and (iii), the following results were obtained:

| Example | Temperature at step (ii) | Temperature at step (iii) | Yield |
|---|---|---|---|
| **1** (above) | 0°C | 25°C | 85% |
| 2 | 0°C | 0°C | 81% |
| 3 | 10°C | 10°C | 82% |
| 4 | 25°C | 25°C | 80% |

### Comparative example 5: Preparation of ethyl 2-(6-formyl-5-methyl-cyclohex-2-en-1-yl)acetate following the procedure described in IN2014DE02082A:

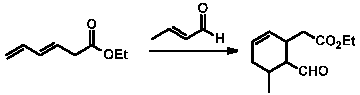

To a solution of ethyl (3*E*)-3,5-hexadienoate (2.5 g, 0.0142 mol) and (*E*)-2-methylbut-2-enal (2.53 g, 0.0362 mol) in dry DCM (67 mL) was added 50% BF₃·OEt₂ solution in diethyl ether (8.14 g, 0.0286 mol) at -78°C dropwise over 20 min. The mixture was allowed to warm to 25°C and was stirred for 2 h at the same temperature. After completion of the reaction, DCM layer was washed with saturated NaHCO₃ (3 x 25 mL) followed by H₂O (25 mL) and brine (25 mL), dried over Na₂SO₄, filtered and concentrated in vacuo to obtain ethyl 2-(6-formyl-5-methyl-cyclohex-2-en-1-yl)-acetate (2.82 g, 75% yield).

### Example 6: Preparation of 8-methyl-1,4,4a,7,8,8a-hexahydroisochromen-3-one

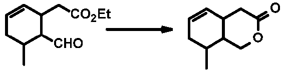

Ethyl 2-(6-formyl-5-methyl-cyclohex-2-en-1-yl) acetate (228 g, 0.952 mol) and MeOH (2000 mL) were mixed at 25°C. The reaction mixture was cooled down to 0°C and a solution of NaBH₄ (54.09 g, 1.43 mol) in NaOH (1 N, 1240 mL) was added dropwise over 60 min. The reaction mass was further stirred at 0°C for 1.5 h. After complete consumption of starting material, the reaction mixture was quenched with HCl (1 N, 2700 ml) to adjust the pH to 3-4 at 0°C (Tr). After quenching, the reaction mixture was warmed to room temperature (RT) and extracted with DCM (2 x 1000 mL). The organic layer was washed with brine (500 ml), dried over sodium sulfate, and evaporated to obtain 8-methyl-1,4,4a,7,8,8a-hexahydroisochromen-3-one (135 g, 85% yield).

*m*/*z* by GCMS 166.

### GCMS Method

| | | | | |
|---|---|---|---|---|
| Total Flow: | 35.4 mL/min | Oven Temp. Program: | | |
| Column Flow: | 1.04 mL/min | Rate (°C/min) | Temp. (°C) | Hold Time (min) |
| Linear Velocity: | 36.8 cm/sec | - | 40.0 | 2.00 |
| Purge Flow: | 3.0 mL/min | 20.00 | 250.0 | 0.00 |
| Split Ratio: | 30.0 | 50.00 | 280.0 | 2.00 |
| Column: | Rxi-5Sil MS, 30m, 0.25mm ID, 0.25µm | | | |

Ethyl 2-(6-formyl-5-methyl-cyclohex-2-en-1-yl) acetate as a mixture of diastereoisomers: 10.17 & 10.25 min. 8-Methyl-1,4,4a,7,8,8a-hexahydroisochromen-3-one as a mixture of diastereoisomers: 10.40 & 10.57 min, m/z 166.

### Example 7: Preparation of 8-methylisochroman-3-one

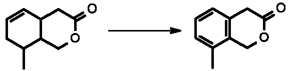

To a magnetically stirred solution of the 8-methyl-1,4,4a,7,8,8a-hexahydroisochromen-3-one (2 g, 12.03 mmol) and Pd/BaSO₄ (200 mg, 10 wt%) in diphenyl ether (10 mL) was added nitrobenzene (741 mg, 6.01 mmol, 50 mol%). The reaction mixture was heated at 150°C under oxygen atmosphere until consumption of starting material. After the completion of reaction, the reaction mixture was cooled to RT, filtered through celite and the celite bed was washed with MeOH. The combined organic layers were concentrated under reduced pressure and purified over silica gel to furnish 8-methylisochroman-3-one (1.08 g, 55% yield).

¹H NMR (300 MHz, CDCl₃): δ 7.28 - 7.24 (q, J = 12 Hz, 1H), 7.15 - 7.13 (d, J = 6.0 Hz, 1H), 7.08 - 7.05 (d, J = 9.0 Hz, 1H), 5.37 (s, 2H), 3.70 (s, 2H), 2.34 (s, 3H).

### GCMS Method

| | | | | |
|---|---|---|---|---|
| Total Flow: | 35.4 mL/min | Oven Temp. Program: | | |
| Column Flow: | 1.04 mL/min | Rate (°C/min) | Temp. (°C) | Hold Time (min) |
| Linear Velocity: | 36.8 cm/sec | - | 40.0 | 2.00 |
| Purge Flow: | 3.0 mL/min | 20.00 | 250.0 | 0.00 |
| Split Ratio: | 30.0 | 50.00 | 280.0 | 2.00 |
| Column: | Rxi-5Sil MS, 30m, 0.25mm ID, 0.25µm | | | |

8-Methyl-1,4,4a,7,8,8a-hexahydroisochromen-3-one as a mixture of diastereoisomers: 10.40 & 10.57 min. 8-Methylisochroman-3-one: 10.81min.

## Claims

1. A process for preparing compounds of formula I 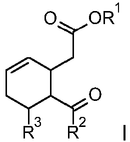 in which
R¹ is H, C₁-C₁₂-alkyl, phenyl, C₁-C₄-alkyl-phenyl, or C₁-C₄-alkyl-C₃-C₆-cycloalkyl, wherein phenyl rings are unsubstituted or substituted with one halogen;
R² is H or C₁-C₆-alkyl; and
R³ is C₁-C₆-alkyl, C₂-C₄-alkenyl, C₃-C₆-cycloalkyl or C₁-C₄-alkoxy, wherein R³ is unsubstituted or substituted with 1, 2, 3, 4 or 5 halogens;
the process comprising:
(i) mixing a compound of formula III 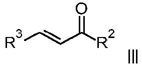
in which R² and R³ are as defined above for formula I,
with a compound of formula II 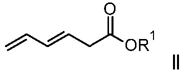
in which R¹ is as defined above for formula I;
(ii) adding boron trifluoride diethyl etherate;
(iii) stirring the mixture;
**characterized by** the fact that the temperature is between -20°C and 80°C.

2. The process of claim 1, in which step (ii) is conducted at a temperature between -5°C and 10°C.

3. The process of any of the claims 1 to 2, in which R¹ is ethyl.

4. The process of any of the claims 1 to 3, in which R² is H.

5. The process of any of the claims 1 to 4, in which R³ is methyl.

6. The process of any of the claims 1 to 5, further comprising reacting the compound of formula I with a reducing agent to yield a compound of formula IV 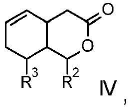 in which R² and R³ are as defined for formula I.

7. The process of claim 6, in which the reducing agent is NaBH₄.

8. The process of any of the claims 6 to 7, further comprising reacting the compound of formula IV with an oxidizing agent to yield a compound of formula V 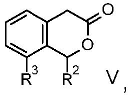 in which R² and R³ are as defined for formula I.

9. The process of claim 8, in which the oxidizing agent comprises Pd/BaSO₄ and O₂.

10. Compounds of formula I 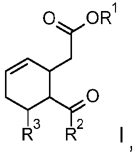
compounds of formula IV 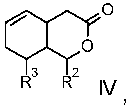
and compounds of formula V 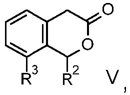
in which R¹, R², and R³ are defined as in any of the claims 1, 3, 4, and 5.

11. The compounds of formula I according to claim 10, wherein R¹ is ethyl.

12. The compounds of formula I, the compounds of formula IV, and the compounds of formula V according to any of the claims 10 or 11 wherein R² is H.

13. The compounds of formula I, the compounds of formula IV, and the compounds of formula V according to any of the claims 10 or 12 wherein R³ is methyl.

14. Use of compounds of formula I 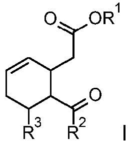
or compounds of formula IV 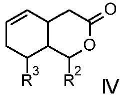
for the preparation of compounds of formula V 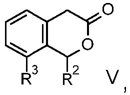
in which R¹, R², R³ are as defined in any of the claims 1, 3, 4, and 5.
